# EUROPEAN PATENT APPLICATION

(11) **EP 3 398 464 A1**
(43) Date of publication of application: **07.11.2018**
(21) Application number: 17169427.6
(22) Date of filing: 04.05.2017
(51) Int. Cl.: A41D 1/00, A61B 5/00, A41D 13/12, A41D 27/08, F21V 8/00

(54) **GARMENT**

(71) Applicant: RPE GmbH, 54329 Konz (DE)
(72) Inventor: The designation of the inventor has not yet been filed
(74) Representative: Hartz, Nikolai

(57) **Abstract**

The present invention provides a process for illuminating a garment (10) worn by an individual, which process comprises the following steps:
(i) providing a garment (10) having an illumination system (20) comprising
(a) an optical fibre member (210) holding fibre ends (212) of multiple optical fibres (211) attached to the garment (10);
(b) a wearable control unit (220) comprising at least one light source (221) releasably attached to the optical fibre member (210), the control unit (220) further comprising a data processor (222), and a wearable power source (223);

(ii) storing sensor data representing context information of the garment (10);
(iii) processing the sensor data so as to generate processed sensor data;
(iv) mapping the processed sensor data to an individual state by using sensor data mapping rules;
(v) mapping the individual state to a light output state by using individual state mapping rules;
(vi) controlling the at least one light source depending on the light output state.

## Description

### Field of the Invention

The present invention relates to a process for illuminating a garment worn by an individual. The present invention also relates to a garment provided with an illumination system adapted for use in the process of the present invention. Furthermore, the present invention relates to a garment comprising a specific optical fibre member. Finally, the present invention relates to an illumination system for a garment.

The present invention provides a fashion electronics platform characterized by defined interfaces between garment, computer hardware, and computer software, which allows separation of design tasks on the garment level, the hardware level, and the software level, and thereby results in enhanced efficiency of the fashion design process for illuminated garments. The fashion electronics platform integrates garments, illumination systems, mobile digital devices, servers, and digital networks in a nonverbal communication model using nonverbal fashion statements reflecting the context of the garment and thereby also the nonverbal context of the wearer of the garment. The mapping of nonverbal contextual cues to an illumination pattern of the garment may be customized to provide nonverbal fashion statements in a new dimension of fashion.

### Background of the Invention

Fashion garments are known. Fashion garments are designed to let the individual wearing the garment make a specific fashion statement. Every season sees a vast number of fashion collections appearing in fashion shows throughout the world. Moreover, the creation of expressive garments by fashion designers also continuously generates a large number of unique garments. Consumers avidly purchase fashion garments, *inter alia* for the potential of making a fashion statement, and often stockpile vast amounts of clothing in closets of their homes. Still, and most surprisingly, consumers are often of the opinion that they do not have anything to wear for the occasion in their closets despite the considerable choice available.

Fashion designers conceive garments by selecting the features of, in particular, the lines, contours, colors, shape, texture and/or materials of the garment itself and/or its ornamentation. The use of electronic components for installation into the garment was mostly alien to a conventional fashion design process in the past.

However, illuminated garments are known. For example, a garment provided with illumination using optical fibres and LEDs is known from http://www.vanityfair.com/style/2016/05/met-gala-2016-red-carpet. Generic garments are one-of-a-kind, hand-sewn creations with electronics adapted to the specific garment, and often the electronic components are at least in part permanently installed on the garment, which requires a close cooperation between the fashion designer and an electronics expert or special knowledge on the part of the fashion designer. Furthermore, the cleaning of the garment is difficult or even impossible when sensitive electronics parts are permanently installed on a garment. Moreover, illumination systems are known which require electrical components to be installed on the human body, which is undesirable for reasons of safety, hygiene and convenience on the part of the consumer.

Moreover, garments responsive to sensor data in real-time are also disclosed in the prior art. The visual impression generated by illuminated garments may range from a technical gadget to a slight enhancement of the conventional design of the garment. A fashion statement adapted to the context of the individual is, however, hardly possible with such conventional illuminated garments.

EP2989974 discloses electric components to be installed into or onto a garment which are provided in/on a module. The module comprises an installation zone via which said module with said components is configured to be installed, such as sewed, quilted, glued, welded or laminated, with the garment in a fixed manner. The installation zone is arranged so that the module is also configured to be detached from the garment via said installation zone by breaking, such as cutting or tearing the structure of said installation zone.

### Summary of the Invention

A need exists for a method to extend the range of possibilities available to a fashion designer designing a garment as well as to an individual wearing the garment to make a fashion statement.

Moreover, a need exists for a fashion electronics platform which enhances the efficiency of a fashion design process for illuminated garments. Furthermore, a fashion electronics platform should allow compatibility of multiple garment designs with one or more control devices. Finally, the platform should preferably include the possibility of downloading and updating software of the control device from a network such as the internet.

Therefore, it is the problem of the present invention to provide a fashion platform which may be adapted to any type of garment for facilitating a fashion dynamically changing fashion statements. More specifically, it is the problem of the present invention to provide a process for illuminating a garment worn by an individual, whereby the context of the individual determines the appearance of the garment, and whereby the process may be adapted for a wide range of garments.

It is a further problem of the present invention to provide a garment provided with an illumination system for use in a process of the present invention, whereby the garment may be cleaned without special precautions in view of any electronic equipment.

It is a further problem of the present invention to provide an illumination system for a garment, which is interactive and responsive and which may be used universally for different garments. The illumination system should use a definite mapping of context to illumination for providing an illumination pattern which is intuitively understood or learned as reflecting specific context.

According to a first aspect, the present invention provides a process for illuminating a garment worn by an individual, which process comprises the following steps:
(i) providing a garment having an illumination system comprising
   (a) an optical fibre member holding fibre ends of multiple optical fibres attached to the garment;
   (b) a wearable control unit comprising at least one light source releasably attached to the optical fibre member, the control unit further comprising a data processor, and a wearable power source;
(ii) storing sensor data representing context information of the garment;
(iii) processing the sensor data so as to generate processed sensor data;
(iv) mapping the processed sensor data to an individual state by using sensor data mapping rules;
(v) mapping the individual state to a light output state by using individual state mapping rules;
(vi) controlling the at least one light source depending on the light output state.

According to a second aspect, the present invention provides a garment provided with an illumination system for use in a process according to the present invention, which comprises an illumination system comprising
(a) an optical fibre member holding fibre ends of multiple optical fibres attached to the garment;
(b) a wearable control unit comprising at least one light source releasably attached to the optical fibre member, the control unit further comprising a data processor, and a wearable power source;
characterized in that the light source comprises an LED matrix having a number of LEDs, and the optical fibre member comprises a plurality of fibres, whereby each fibre is illuminated by a specific LED of the light source when the at least one light source is releasably attached to the optical fibre member.

According to the third aspect, the present invention provides a garment comprising an optical fibre member holding fibre ends of at least two optical fibre bundles attached to the garment, wherein optical fibre member is a plastic shell holding and arranging the optical fibre bundles as a n x m matrix, wherein each fibre bundle is independently composed of at least 3 optical fibres, and wherein n is an integer of 1 to 10 and m is an integer of from 2 to 10, so that the fibers are exposed to the inside of the shell for allowing light to enter the fibres.

According to a fourth aspect, the present invention provides an illumination system for a garment (10), which comprises
(a) an optical fibre member holding fibre ends of one or more optical fibre bundles to be attached to the garment;
(b) a wearable control unit comprising at least one light source releasably attached to the optical fibre member, the control unit further comprising a data processor, and a wearable power source, wherein the wearable control unit further comprises
   (b-1) a data collector configured to collect and store sensor data;
   (b-2) a data processor configured to extract processed sensor data from the sensor data; and
   (b-3) an individual state determiner configured to provide the processed sensor data to an inference model to determine the individual state based on the processed sensor data by using individual state rules.

The present invention starts out by the assumption that the subjective belief that the fully stuffed closet does not contain anything to wear for the occasion in fact means that the closet does not contain any garment suitable for making the fashion statement desired for the occasion. Moreover, the present invention is based on the recognition that static illumination of a garment provides a single less desirable fashion statement, whereas a simple dynamic illumination of a garment may provide a fashion statement which is undesirable in a given moment. However, a dynamic illumination which is responsive to the context of the individual wearing the garment, is able to provide fashion statements which are in line with the context of the individual and, therefore, subjectively provide the experience that changing, but appropriate fashion statements are made by the garment, and therefore allows an interactive use of the garment.

According to a first aspect, the present invention is based on the concept of mapping the context information of the garment to an expressive light pattern reflecting the context of the individual in real-time.

According to a second aspect, the present invention is further based on the concept of providing a fashion electronics platform characterized by defined interfaces between the garment, the hardware, and, preferably, the software, whereby compatibility of the components of the fashion electronics platform allows separation of design tasks on the garment level, the hardware level, and the software level.

According to a third aspect, the present invention is further based on the concept that a specific optical fibre member may be used for designing a garment based on conventional designing techniques whereby indirect illumination by using optical fibres provides a more esthetic effect as compared to plain LED lights. Moreover, the optical fibre member as such does not require any electronics so that the garment may be washed or dry-cleaned without damaging the wearable control unit.

According to a fourth aspect, the present invention is further based on the concept of providing an illumination system which comprises two parts which may be independently designed, and which may be combined in order to provide illumination of the garment.

The aspects of the present invention are parts of a fashion electronics platform. The features of each aspect described herein may be combined with any other aspect of the present invention.

### Description of the Figures

Fig. 1 shows a flow chart of the process of the present invention.
Fig. 2 shows a schematic front view of a garment such as a skirt comprising an illumination system according to the present invention.
Fig. 3 shows a schematic view of a garment such as a skirt comprising an optical fibre member attached according to the present invention.
Fig. 4 shows a schematic front view of the illumination system of the garment according to the present invention. The light source forming part of the wearable control unit is covered by the optical fibre member and, therefore, not visible in this view.
Fig. 5 shows a schematic front view of a wearable control unit according to the present invention, including a light source, a data processor and a wearable power source.
Fig. 6 shows a schematic view of an optical fibre member according to the present invention.
Fig. 7 shows a detailed schematic front view of the wearable control unit shown in Fig. 4.
Fig. 8 shows a schematic view of a garment such as a skirt comprising an illumination system including a wearable control unit having a wireless communication module and the mobile digital device also having a wireless communication module. The mobile digital device is connected to a network such as the internet for communication with a server.
Fig. 9 shows a detailed schematic front view of a wearable control unit having a wireless communication module and a mobile digital device having a wireless communication module connected to a server through a network.

### Detailed Description of the Invention

### Definitions

The term "fashion statement" refers to nonverbal cues conveyed by garments in order to attract attention and to show other people the type of person the wearer of the garment is, not necessarily in a bold way. A fashion statement can be used according to the present invention to attract the attention of others or can demonstrate mood, level of confidence, interests, culture, age, and values/beliefs.

The term "context" refers to a collection of interrelated conditions in which the garment exists. When the garment is worn by an individual, the context of the garment merges at least in part with the context of the individual. The term "context information" refers to a representation of context, such that it can be communicated in a system, including applications.

The term "module" may refer to a unit including one of hardware, software, and firmware or a combination of two or all of them. The term "module" may be interchangeably used with, for example, the term "unit", "logic", "component", or "circuit". The "module" may be a minimum unit of an integrated component element or a part thereof. The "module" may also be a minimum unit for performing one or more functions or a part thereof. The "module" may be mechanically or electronically implemented.

When an element is referred to as being operatively or functionally "connected," or "coupled," to another element, it may be directly connected or coupled directly to the other element, or any other element may be interposed between them as long as the subject connection or coupling is operative or functional. When an element is referred to as being "directly connected" or "directly coupled" to another element, there is no element interposed between them.

The expression "configured to" used in the present disclosure may be exchanged with, for example, "suitable for", "having the capacity to", "designed to", "adapted to", "made to", or "capable of" according to the situation. The term "configured to" may not necessarily imply "specifically designed to" in hardware. Alternatively, in some situations, the expression "device configured to" may mean that the device, together with other devices or components, "is able to". For example, the phrase "processor adapted (or configured) to perform A, B, and C" may mean a dedicated processor (e.g. embedded processor) only for performing the corresponding operations or a generic-purpose processor (e.g., central processing unit (CPU) or application processor (AP)) that can perform the corresponding operations by executing one or more software programs stored in a memory device.

### The process according to the present invention

According to the first aspect, the present invention provides a process for illuminating a garment worn by an individual. The illumination may be directed to all or a part of the garment. All of the garment may be illuminated by distributing and attaching optical fibres all over the garment at about the same density. A part of the garment may be illuminated by distributing and attaching optical fibres with preference to certain parts of the garment. For examples, all of a skirt may be illuminated by distributing and attaching the optical fibres on the front, the sides and the back of the skirt. A dress may be illuminated in part by distributing and attaching the optical fibres only at the lower portion of the dress.

The process of the present invention provides illumination which is dynamic and responsive. The term "dynamic" means with regard to the process of the present invention that the illumination changes as a function of time. Therefore, the nonverbal cues which are conveyed by the garment change as a function of time. The changes may relate to hue, saturation and brightness, and any combination thereof. Preferably, the changes relate to a combination of the brightness and hue. The changes may be implemented by using an RGB LED based on a RGB color model. The RGB color model describes a color by using three variables, Red, Green and Blue. These variables provide an additive color model when the three color components together form a color.

The term "responsive" means with regard to the process of the present invention that the illumination dynamically reflects the changing context of the individual wearing the garment. Therefore, the nonverbal cues conveyed by the garment take the context into account.

The illumination is designed to enhance and multiply the fashion statements available with the garment. Preferably, the fashion statement is determined based on the context of the individual so that the state of the individual, including movement, proximity, emotions, and the like is reflected by the illumination of the garment.

The type of garment is not particularly limited and may be selected from any conventional types of garments. Examples for garments are skirts, dresses, shirts, coats, suits, and jackets. Preferably, the garment is a skirt or dress.

The textile of the garments is not particularly limited and may be preferably selected from a textile which comprises a netting. Preferably, the textile of the garment has properties such as transparence for or reflectance or scattering of the light of the illumination system. Preferably the garment comprises multiple layers of material.

The garment may the adapted for artistic performances such as an entertainment show. The garment may also be adapted as a party dress or evening dress. Preferably, the garment is worn in a dark environment so that the effect of the illumination is easily visible.

The individual wearing the garment may be male or female.

The process for illuminating a garment worn by an individual according to the present invention comprises a step of providing the garment having an illumination system. The illumination system comprises two separate parts which are releasably attached to each other. A first part may preferably be kept attached to the garment during washing or dry cleaning. A second part may be separated from the garment for charging of the power source or combination with another garment.

The illumination system comprises an optical fibre member for holding multiple optical fibre ends. The optical fibre member comprises optical fibres attached to the garment. Preferably, the optical fibre ends are arranged in bundles. The optical fibres serve as design elements on the garment level. The optical fibres preferably are positioned so that the fibre end opposite to the end held by the optical fibre member, provides a light point on the garment so that the fibre end opposite to the end held by the optical fibre member will emit light at a predetermined position on the garment. Additionally, the cladding of the optical fibre may be cut so that light leaches out of the side of the fibre, which further provides a design option.

A suitable optical fibre is a flexible, transparent fiber made by drawing, for example, plastic or glass. Preferably, the diameter of an optical fibre is preferably in the range of from 0.1 to 1.5 mm, more preferably 0.2 to 0.9 mm, still more preferably 0.3 to 0.8 mm. The optical fibres attached to the garment may have the same length or different length. Preferably, the length of the fibres is in the range of from 20 to 120 cm, more preferably 50 to 90 cm depending on the type and size of the garment. Suitable optical fibres typically include a transparent core surrounded by a cladding material with a lower index of refraction. Light is kept in the core by total internal reflection which causes the fibre to act as a waveguide. Accordingly, an optical fibre useful in the present invention is preferably a cylindrical dielectric waveguide that transmits light along its axis. The boundary between the core and cladding may either be abrupt, in step-index fiber, or gradual, in graded-index fiber. According to a preferred embodiment, the cladding may be locally removed in order to allow light to escape from the lateral surface of the fibre.

The material of the optical fibres is not particularly limited. Plastic optical fibers (POF) are preferred for reasons of cost. However, quartz or glass optical fibres made from silica, or other materials, such as fluorozirconate, fluoroaluminate, and chalcogenide glasses as well as crystalline materials like sapphire, may also be used. Plastic optical fibers may comprise polymethylmethcrylate, polystyrene or polycarbonate, for example.

Light generated by the illumination system is transmitted into the fibres through fibre ends. The optical fibre member serves to orient and immobilize the optical fibre ends. The plurality of optical fibres may be bundled together and encased in a socket for attachment to the optical fibre member. Alternatively, the individual fibres may be braided into a fibre ribbon. The ribbon may have a flat or circular cross-section.

The illumination system further comprises a wearable control unit comprising at least one light source releasably attached to the optical fibre member. The control unit further comprising a data processor, and a wearable power source. The data processor controls, in particular, the light source. The power source provides electrical power for the light source and the data processor. According to a preferred embodiment, the wearable control unit further comprises one or more sensors.

The process for illuminating a garment worn by an individual according to the present invention further comprises a step of storing sensor data representing context information of the garment. The sensor data may be data generated by one or more sensor modules. The sensor modules are preferably logging the context of the garment. Suitable sensor data may be derived from one or more sensor modules selected from one or more sensor types including accelerometer sensors, gyroscope sensors, distance sensors, magnetometer sensors, orientation sensors, global positioning systems, conductivity sensors, illuminance sensors, and microphones. The sensor modules may be part of the wearable control unit. Alternatively, the sensors may be part of a mobile digital device such as a smartphone. The sensor data representing context information of the garment may be stored by a specific module such as a data collector configured to collect and store sensor data.

The process for illuminating a garment worn by an individual according to the present invention further comprises a step of processing the sensor data so as to generate processed sensor data. The sensor data representing context information of the individual obtained from the sensors is raw data. In order to improve the quality of the raw data, the data is processed. The type of processing is not particularly limited as long as the quality of the raw data is improved, for example by reducing noise, and preferably the size of the data is reduced. It is preferable that discretization of continuous sensory data is performed. Furthermore, data profiling techniques may be used for analyzing the data for correctness, completeness, uniqueness, consistency, and reasonability. The data may also be subjected to data cleansing for detecting and correcting (or removing) corrupt or inaccurate data and to identify incomplete, incorrect, inaccurate or irrelevant parts of the data and then replacing, modifying, or deleting the dirty or coarse data. As a result, processed sensor data are obtained. The processed sensor data may be obtained by specific module such as a data processor configured to extract processed sensor data from the sensor data. The data processor may preferably perform discretization of continuous sensory data.

The process for illuminating a garment worn by an individual according to the present invention further comprises a step of mapping the processed sensor data to an individual state by using sensor data mapping rules. The mapping of the processed sensor data to an individual state may be implemented by a specific module such as an individual state determiner configured to provide the processed sensor data to an inference model to determine the individual state based on the processed sensor data by using individual state rules. The individual state determiner may include classifying rules provided as hidden Markov models and/or Neuronal Networks and/or Gaussian Mixture Models and/or decision trees.

The sensor data mapping rules may be defined according to any method suitable for interpreting the processed sensor data. Preferably, the sensor data mapping rules are determined by a machine learning algorithm. Attribute selection depends on the available sensor data. Based on labeled training data, sensor data mapping rules may, preferably, be selected based on supervised learning techniques. Any supervised classification algorithms may be used for deriving sensor data mapping rules from labeled training data. As an example, decision tree (for example J48), JRib, or Naïve Bayes may be mentioned.

The process for illuminating a garment worn by an individual according to the present invention further comprises a step of mapping the individual state to a light output state by using individual state mapping rules. Individual state mapping rules may be implemented as a finite state machine by defining a list of its light output states, an initial state, and the conditions for each transition based on the individual states. Accordingly, the garment may be in exactly one of a finite number of light output states at any given time. Transitions from one light output state to another occur in response to changes of the individual state.

The process for illuminating a garment worn by an individual according to the present invention further comprises a step of controlling the at least one light source depending on the light output state. The at least one light source may be controlled by any suitable method. Preferably, the control of the one or more light source is adapted to the context of the individual. For example, when the individual is active, the one or more light sources could be controlled so as to provide an active and bright illumination pattern. Similarly, when the individual is passive, the one or more light sources could be controlled so as to provide a passive and less bright illumination pattern.

Preferably, in the process according to the first aspect, the wearable control unit further comprises a first wireless communication module and the sensor data mapping rules and/or the individual state mapping rules are stored in a mobile digital device having a second wireless communication module connected to the wearable control unit by a wireless communication channel, and preferably, wherein the sensor data mapping rules and/or the individual state mapping rules stored in the mobile device are downloaded from a database connected to the internet.

According to a preferred embodiment, sensor data mapping rules are stored on a mobile digital device. Accordingly, the wearable control unit requires a wireless communication channel to the mobile digital device in order to control the at least one light source depending on the light output state. A mobile digital device according to the present invention may be selected from a smart phone, a tablet personal computer, a mobile phone, a video phone, an electronic book reader, a netbook computer, digital assistant (PDA), or a portable multimedia player (PMP). Preferably, the mobile digital device is a smart phone.

A mobile digital device within a network environment may include a bus, a processor, a memory, an input/output interface, a display, and a wireless communication module. At least one of the above components may be omitted or additional components may be included. The wireless communication module may establish communication between the mobile digital device and the wearable control unit. Moreover, the wireless communication module or an additional module may establish communication between the mobile digital device and a server in a network such as the internet.

The wireless communication module of the mobile digital device may be connected to a network through wireless communication, and may communicate with the wearable control unit or the server. The wireless communication may use at least one of, for example, short range communication. The short-range communication may include at least one of, for example, Bluetooth, Wi-Fi, and near field communication (NFC).

The wireless communication of the mobile digital device may also use at least one of, for example, long range communication, such as long term evolution (LTE), LTE-advance (LTE-A), code division multiple access (CDMA), wideband CDMA (WCDMA), universal mobile telecommunications system (UMTS), wireless broadband (WiBro), and global system for mobile communications (GSM), as a cellular communication protocol. In addition, the network may include at least one of a telecommunication network such as a computer network (e.g., a local area network (LAN) or a wide-area network (WAN)), the Internet, and a telephone network.

According to a first alternative, the sensor data are collected by the mobile digital device from one or more sensors of the mobile digital device. Accordingly, the mobile digital device must be worn by the individual so that relevant context information of the individual may be collected by the device. Moreover, the sensor data are preferably processed by the mobile digital device so as to generate processed sensor data which are then preferably stored on the mobile digital device. Subsequently, the processed sensor data is preferably mapped to an individual state by using sensor data mapping rules stored on the mobile digital device. The individual state may be communicated to the wearable control unit for mapping the individual state to a light output state by using individual state mapping rules stored on the wearable control unit. Alternatively, the individual state is mapped to a light output state by using individual state mapping rules stored on the mobile digital device and the light output state is communicated to the wearable control unit. Finally, the wearable control unit controls the at least one light source depending on the light output state.

According to a second alternative, the sensor data are collected by wearable control unit from one or more sensors attached to the wearable control unit. Accordingly, the one or more sensors are attached to the garment worn by the individual so that relevant context information of the individual may be collected by the wearable control unit. Subsequently, the sensor data are processed by the wearable control unit so as to generate processed sensor data which are then stored on the mobile digital device. Subsequently, the processed sensor data are mapped to an individual state by using sensor data mapping rules stored on the mobile digital device. For this purpose, sensor data mapping rules are communicated from the mobile digital device to the wearable control unit. The individual state may then be mapped to a light output state by using individual state mapping rules stored on the wearable control unit. Alternatively, the individual state may then be mapped to a light output state by using individual state mapping rules stored on the mobile digital device. For this purpose, either the individual state is communicated to the mobile device for mapping by using individual state mapping rules stored on the mobile digital device, or the individual state mapping rules stored on the mobile digital device are communicated to the wearable control unit for mapping.

Preferably, in the process according to the first aspect, the system comprises an individual state determiner configured to provide the processed sensor data to an inference model to determine the individual state. The individual state determiner may include classifying rules provided as hidden Markov models and/or Neuronal Networks and/or Gaussian Mixture Models and/or decision trees. Preferably, the inference model is based on a supervised learning algorithm, wherein the supervised learning algorithm preferably provides a decision tree algorithm or a naive Bayes classification algorithm. According to a preferred embodiment, the sensor data mapping rules are determined by a machine learning algorithm. According to a specific embodiment of the process according to the first aspect, the individual state is used as the light output state.

Preferably, in the process according to the first aspect, the wearable control unit and/or the mobile device further comprises one or more sensors selected from an accelerometer sensor, a gyroscope sensor, a distance sensor, a magnetometer sensor, an orientation sensor, a global positioning system, GPS, a conductivity sensor, an illuminance sensor, and a microphone for providing sensor data representing context information of the individual.

Preferably, in the process according to the first aspect, the light source is an LED matrix or a OLED screen, which emit light of a wavelength in the range of from 350 to 850 nm.

Preferably, in the process according to the first aspect, the wireless communication module of the mobile device connects to the wireless communication module of the control unit by using a Bluetooth protocol.

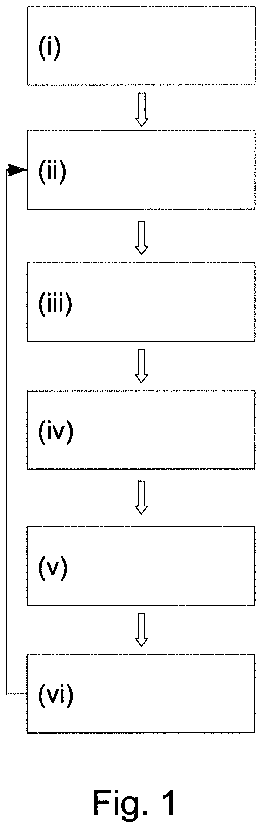

Fig. 1 shows a flow chart of the process of the present invention. Accordingly, in a first step (i), a garment having an illumination system is provided. In a second step (ii), sensor data representing context information of the garment is stored. In a third step (iii), the sensor data is processed so as to generate processed sensor data. In a fourth step (iv), the processed sensor data is mapped to an individual state by using sensor data mapping rules. In a fifth step, (v) the individual state is mapped to a light output state by using individual state mapping rules. According to the sixth step (vi), the at least one light source is controlled depending on the light output state. Steps (ii) to (vi) may be repeated in a loop while the garment is worn by an individual.

### The garment according to the present invention

According to the second aspect, the present invention also provides a garment for use in a process according to the first aspect.

According to a preferred embodiment, the garment is women apparel selected from a dress and a skirt. Preferably, the garment comprises a netting such as a bobbinet, tulle, fishnet, filet, or maline. Bobbinet is a hexagonal mesh that is preferably thin and transparent by selecting a suitable yarn count of the material used to produce it. Tulle is a lightweight, very fine netting which can be made of various fibres, including silk, nylon, and rayon. The netting is preferably transparent, reflective and/or scatters the light from the illumination system. Moreover, a netting may be advantageously used for the purposes of the present invention for holding optical fibres. Preferably, the garment comprises multiple layers of net such as bobbinet or tulle. Accordingly, a preferred garment is based on a layered tulle skirt or dress.

The garment comprises an illumination system. The illumination system comprises an optical fibre member and a wearable control unit.

Preferably, the optical fibre member does not contain any electronic parts including wiring. Preferably, the optical fibre member consists essentially of dielectric material, more preferably polymer. Moreover, the optical fibre member may be permanently or releasably attached to the garment. According to a preferred embodiment, the optical fibre member may remain attached to the garment when the garment is washed or dry-cleaned. Alternatively, the optical fibre member may be separated from the garment for cleaning. The optical fibres are preferably interlaced in the netting.

The optical fibre member preferably comprises bundles of optical fibres. The bundles contain preferably 2 to 20 fibres depending on the diameter of each optical fibre. An increase of the number of optical fibres results in an increase of the rigidity of the fibre bundle. For a fibre diameter of from 0.5 to 1.0 mm, each bundle preferably contains 3 to 10 fibres, more preferably, 4 to 8 optical fibres. The optical fibres in a bundle may have the same length. Typically, the length may be selected from the range of from 20 cm to 110 cm. Alternatively, the optical fibres of a bundle may have different length. The optical fibres in a bundle may have the same diameter. Alternatively, the optical fibres of a bundle may have different diameter. In case multiple bundles are present, the bundles may be the same or different.

The properties of the optical fibres are similar to the properties of fibres conventionally used in fashion design. Therefore, the fashion design process of the garment according to the present invention may use conventional fashion design techniques subject to the requirement that the optical fibre member holding the optical fibre ends may be releasably attached to the wearable control unit.

The optical fibre member holding fibre ends represents an origin of the light generated by the illumination system. Given the high density of fiber ends, the optical fibre member holding fibre ends may be the brightest portion of the garment, and, therefore, much visible. Therefore, the position of the optical fibre member on the garment may be deliberately chosen as a matter of fashion design. The specific position of the optical fibre member on the garment is not particularly limited. Suitable positions on are, for example, the waist, shoulder or chest.

Preferably, the wearable control unit comprises at least one light source releasably attached to the optical fibre member. The light source and the optical fibre member preferably form a hollow space in between when attached to each other. The hollow space serves as a housing for one or more LEDs or OLED. The optical fibre member, preferably, has openings in which the optical fibre bundles are held and arranged so that the fibre ends are in close proximity to the light source. According to a preferred embodiment, the wearable control unit is held by the optical fibre member, and is not permanently attached to the garment. However, it is also preferred that all or a part of the wearable control unit is held in a pocket or the like provides on the garment or an accessory such as a belt.

According to a preferred embodiment, the light source comprises one or more LED matrices having a number of LEDs, and the optical fibre member comprises a plurality of fibres, whereby each fibre is illuminated by a specific LED of the light source when the at least one light source is releasably attached to the optical fibre member. An LED matrix may be any rectangular array of LEDs, for example, up to a 20x20 LED matrix. Preferably, a 8x1 matrix, a 8x4, or 8x8 matrix. Preferably, in the garment according to the second aspect, an LED of the LED matrix illuminates a bundle of optical fibres.

In the garment according to the second aspect, the control unit further comprises a data processor, and a wearable power source. Preferably, the wearable control unit further comprises one or more sensor modules selected from an accelerometer sensor module, a gyroscope sensor module, a distance sensor module, a magnetometer sensor module, an orientation sensor module, a global positioning system module, a conductivity sensor module, an illuminance sensor module, and a microphone module.

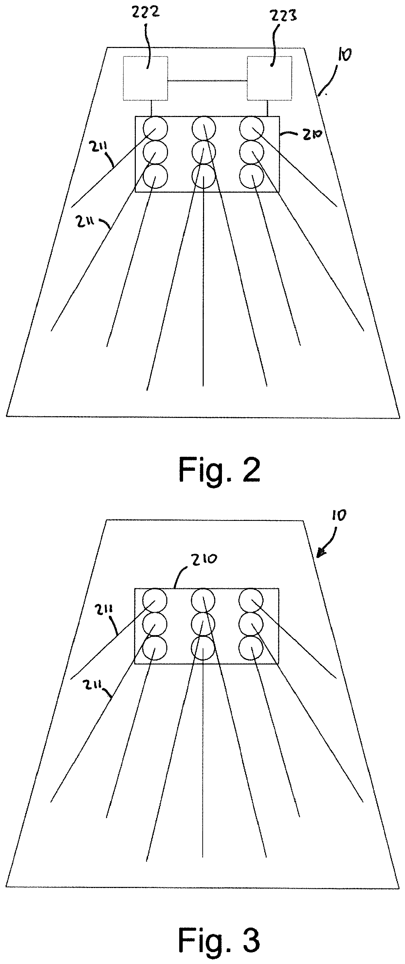

Fig. 2 shows a schematic front view of a garment 10 such as a skirt comprising an illumination system according to the present invention. The illumination system 20 comprises an optical fibre member 210 holding fibre ends 212 of multiple optical fibres 211 attached to the garment 10. The illumination system further comprises a wearable control unit 220 comprising at least one light source 221 releasably attached to the optical fibre member 210. Furthermore, the control unit 220 further comprising a data processor 222, and a wearable power source 223.

According to the third aspect, the present invention also provides a garment comprising an optical fibre member holding fibre ends of at least two optical fibre bundles attached to the garment. Preferably, the optical fibre member holding fibre ends of at least 8 and up to 64 optical fibre bundles.

The optical fibre member according to the fourth aspect is a plastic shell having a convex outer shape. Preferably, the optical fibre member is made of a material which is intransparent to the illumination light so that light is guided only through the optical fibres out of the inside of the space formed by the plastic shell covering the light source. The process for preparing the plastic shell is not particularly limited, and injection moulding or 3D-printing may be mentioned.

The optical fibre member holds and arranges the optical fibre bundles preferably as a n x m matrix, wherein n is an integer of 1 to 10 and m is an integer of from 2 to 10. Each fibre bundle is independently composed of at least 3 optical fibres whereby the ends of the fibres are exposed to the inside of the shell for allowing light to enter the fibres.

Fig. 3 shows a schematic view of a garment such as a skirt comprising an optical fibre member attached according to the present invention. Specifically, Fig. 3 shows a garment 10 comprising an optical fibre member 210 holding fibre ends 212 of at least two optical fibre bundles attached to the garment 210, wherein optical fibre member 210 is a plastic shell holding and arranging the optical fibre bundles as a n x m matrix, wherein each fibre bundle is independently composed of at least 3 optical fibres 211, and wherein n is an integer of 1 to 10 and m is an integer of from 2 to 10, so that the fibers are exposed to the inside of the shell for allowing light to enter the fibres 211.

### The illumination system

According to the fourth aspect, the present invention provides an illumination system for a garment.

The illumination system according to the present invention preferably is adapted to function as a context aware device useful for the acquisition of context (by using sensors to perceive a situation), understanding of the context (by matching a perceived sensory stimulus to a context), and adapting illumination based on the recognized context (e.g., light output based on context). The illumination system preferably adapts the behavior, makes decisions, or changes illumination without requiring explicit user inputs.

The illumination system comprises at least two physically separable parts. Specifically, as a first part the illumination system comprises an optical fibre member holding multiple optical fibre ends of optical fibres to be attached to the garment. Preferably, the optical fibre ends are arranged in bundles.

The optical fibre member may be plastic shell adapted to cover an LED light source, for example an LED matrix, whereby the optical fibre ends held by the plastic shell are oriented and positioned in close proximity of each of the LEDs of the LED light source. Preferably, the plastic shell is essentially intransparent to the light of the LEDs so that illumination of the garment is essentially provided only by light guided and irradiated by optical fibres. The material of the plastic shell is not particularly limited as long as the dimensional stability of the shell is not compromised by the heat generated by the LED.

The illumination system further comprises a wearable control unit comprising at least one light source releasably attached to the optical fibre member, the control unit further comprising a data processor, and a wearable power source.

Although illumination by LED light tends to be esthetically less advantageous, the light source according to the present invention preferably comprises an LED. Given that the light is distributed and irradiated by intermediate optical fibres, the LED light may be rendered softer and more esthetically pleasing. More preferably, the light source comprises multiple LEDs. An examples of a suitable LED is an intelligent control LED light source such as a WS2812 LED which is an RGB LED with a WS2811 control IC built into the LED. The light source should be selected so that the power consumption is sufficiently low so that the illumination system may be used for an extended period of time of at least 3 hours by using a light-weight battery having from 300 to about 3000 mAh.

The data processor may be based on a microcontroller. Suitable microcontrollers include an Advanced RISC Machine (ARM) or 8051 (Intel MCS-51) core, for example megaAVR microcontrollers, such as an Atmel® picoPower® ATmega328/P which is a low-power CMOS 8-bit microcontroller based on the AVR® enhanced RISC architecture. Atmel ATmega48, ATmega88, ATmega 168, or ATmega 168 are further examples of useful 8-bit microcontroller containing an arithmetic logic unit, flash memory, and I/O functions. More powerful microcontrollers are also suitable for the purposes of the present invention provided that the power consumption of the microcontroller is sufficiently low so as to allow the use of the illumination system for an extended period of time of at least 3 hours by using a light-weight battery.

A wearable control unit contains a battery module as a power source providing power for the light source, the microcontroller and sensors. The power source may be any power source providing at sufficient power to operate the illumination system for at least 3 hours. Preferably, light-weight battery having from 300 to about 10000 mAh may be used.

According to a preferred embodiment, the illumination system comprises one or more sensors functionally connected to the data processor. The sensor may be any sensor module useful for generating context information of the garment. According to a preferred embodiment, the illumination system comprises a distance sensor module. Alternatively or additionally, the illumination system may further comprise a motion sensor module. The motion sensor module may include an accelerometer and a gyroscope.

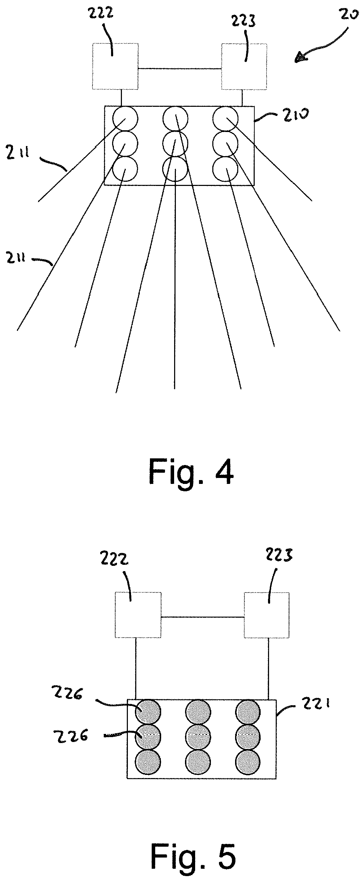

Fig. 4 shows in a schematic front view of the illumination system of the garment according to the present invention. The light source forming part of the wearable control unit is covered by the optical fibre member and, therefore, not visible in this view. Specifically, Fig. 4 shows an illumination system 20 for a garment 10, which comprises an optical fibre member 210 holding fibre ends 212 of one or more optical fibre bundles to be attached to the garment 10. The illumination system 20 for a garment 10 further comprises a wearable control unit 220 comprising at least one light source 221 releasably attached to the optical fibre member 210, the control unit 220 further comprising a data processor 222, and a wearable power source 223. The wearable control unit 220 further comprises a data collector configured to collect and store sensor data. The wearable control unit 220 further comprises a data processor configured to extract processed sensor data from the sensor data. The wearable control unit 220 further comprises an individual state determiner configured to provide the processed sensor data to an inference model to determine the individual state based on the processed sensor data by using individual state rules, preferably further comprising a wireless communication module.

The sensor module 225 may include at least one of a gesture sensor, a gyro sensor, an atmospheric pressure sensor (barometer), a magnetic sensor, an acceleration sensor, a grip sensor, a proximity sensor, a color sensor (e.g., red, green, and blue (RGB) sensor), a biometric sensor (medical sensor), a temperature/humidity sensor, an illuminance sensor, and a ultra violet (UV) sensor. The sensor module 225 may further include a control circuit for controlling one or more sensors included therein. The wearable control unit further includes a processor configured to control the sensor module 225, as a part of the data processor 222 or separately from the data processor 222, and may control the sensor module 225 while the processor 222 is in a sleep state.

The sensors allow the illumination system to monitor the garment and the environment and to infer a model of the current context of the wearer. Such a context model can include a variety of attributes that represent information about the wearer and the wearer's environment. For example, information about the wearer can include raw physiological data (e.g., heart rate) and geographic information (e.g., location and speed), or may attempt to characterize or predict the wearer's physical activity (e.g., dancing or walking), emotional state (e.g., excitement). Background information can also be included, such as the wearer's age or gender. The model can similarly hold environment information, such as air temperature or raw data from a motion sensor, or the distance of nearby people or objects. The model of the current context can additionally include information added explicitly from other sources, as well as user-specified or system-learned defaults and preference information.

The at least one light source 221 of the wearable control unit 220 is releasably attached to the optical fibre member 210. Accordingly, the wearable control unit 220 may be detached from the optical fibre member 210 for washing or dry cleaning of the garment. The optical fiber member 210 may be attached to a light source 221, for example, by a positive-fit connection. Preferably, the attachment is secured, for example, by one or more clips or the like.

The illumination system 20 according to the present invention may be connected to one or more networks 500 of other devices preferably through wireless communication means. In general, as used herein, communications or data exchange between the devices or components described herein may preferably be performed using any wireless methods, including Bluetooth, wireless RF, wireless satellite connections, a cellular phone.

The wearable control unit according to the present invention further comprises a data collector configured to collect and store sensor data.

The wearable control unit according to the present invention further comprises a data processor configured to extract processed sensor data from the sensor data.

The wearable control unit according to the present invention further comprises an individual state determiner configured to provide the processed sensor data to an inference model to determine the individual state based on the processed sensor data by using individual state rules.

Fig. 5 shows a schematic front view of a wearable control unit according to the present invention, including a light source 221, a data processor 222 and a wearable power source 223.

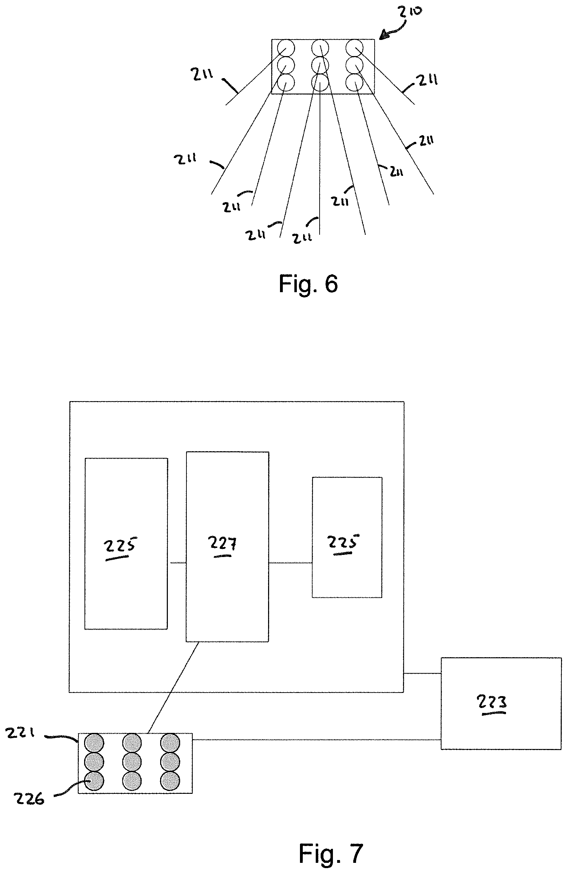

Fig. 6 shows a schematic view of an optical fibre member 210 according to the present invention, which is a plastic shell having optical fibres attached in a 3 x 3 matrix arrangement. The fibre ends are held in a socket attached in an orifice of the plastic shell so that the fibre ends protrude through the wall of the plastic shell.

Fig. 7 shows a detailed schematic front view of the wearable control unit 220 shown in Fig. 5. The wearable control unit comprises sensors operably connected with the data processor.

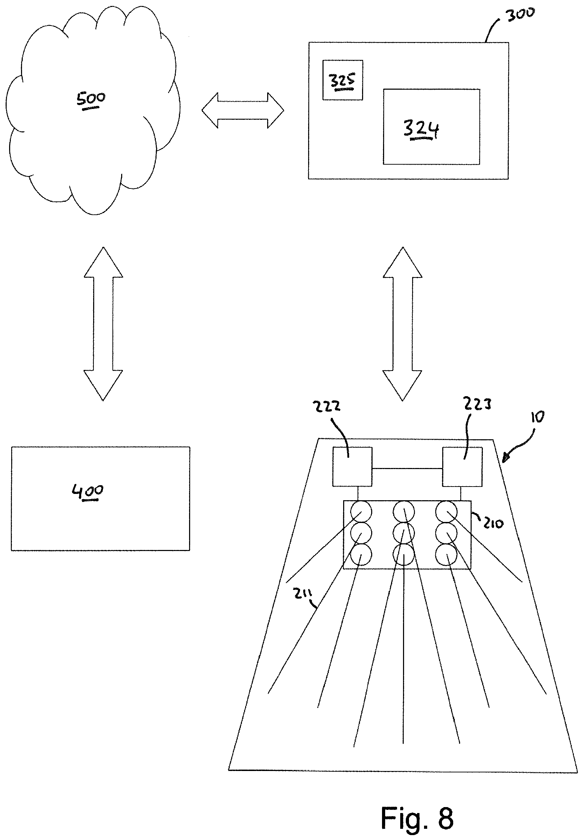

Fig. 8 shows a schematic view of a garment 10 such as a skirt comprising an illumination system 20 including a wearable control unit 220 having a wireless communication module 224 and the mobile digital device 300 also having a wireless communication module324. The mobile digital device 300 is connected to a network 500 such as the internet for communication with a server 40.

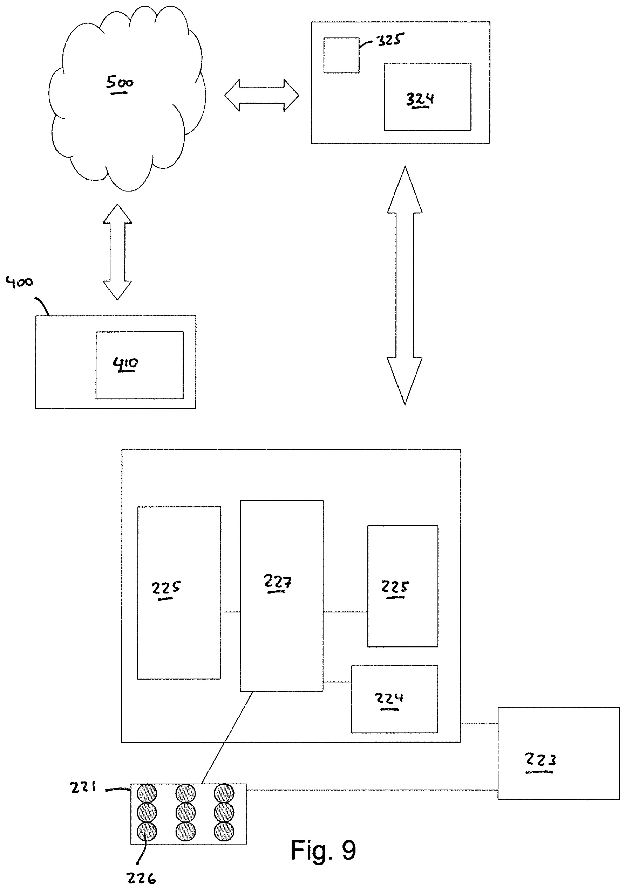

Fig. 9 shows a detailed schematic front view of a wearable control unit 220 having a wireless communication module 224 and a mobile digital device 300 having a wireless communication module 324 connected to a server 40 through a network 500. The wearable control unit further comprises sensors operably connected to the data processor.

## Claims

1. A process for illuminating a garment (10) worn by an individual, which process comprises the following steps:
(i) providing a garment (10) having an illumination system (20) comprising
(a) an optical fibre member (210) holding fibre ends (212) of multiple optical fibres (211) attached to the garment (10);
(b) a wearable control unit (220) comprising at least one light source (221) releasably attached to the optical fibre member (210), the control unit (220) further comprising a data processor (222), and a wearable power source (223);
(ii) storing sensor data representing context information of the garment (10);
(iii) processing the sensor data so as to generate processed sensor data;
(iv) mapping the processed sensor data to an individual state by using sensor data mapping rules;
(v) mapping the individual state to a light output state by using individual state mapping rules;
(vi) controlling the at least one light source depending on the light output state.

2. The process according to claim 1, wherein the wearable control unit (220) further comprises a first wireless communication module (224) and wherein sensor data mapping rules and/or individual state mapping rules are stored in a mobile digital device (300) having a second wireless communication module (324) connected to the wearable control unit (220) by a wireless communication channel, and preferably, wherein the sensor data mapping rules and/or the individual state mapping rules stored in the mobile device are downloaded from a database (410) connected to the internet (500).

3. The process according to claim 1 or 2, wherein the system comprises an individual state determiner configured to provide the processed sensor data to an inference model to determine the individual state.

4. The process according to claim 3, wherein the inference model is based on a supervised learning algorithm, and preferably, wherein the supervised learning algorithm comprises a decision tree algorithm or a naive Bayes classification algorithm.

5. The process according to any one of the preceding claims, wherein the sensor data mapping rules are determined by a machine learning algorithm.

6. The process according to any one of the preceding claims, wherein the individual state is used as the light output state.

7. The process according to any one of the preceding claims, wherein the wearable control unit and/or the mobile device further comprises one or more sensors (225, 325) selected from an accelerometer sensor, a gyroscope sensor, a distance sensor, a magnetometer sensor, an orientation sensor, a global positioning system, a conductivity sensor, an illuminance sensor, and a microphone for providing sensor data representing context information of the garment.

8. The process according to any one of the preceding claims, wherein the light source is an LED matrix or a OLED screen, which emit light of a wavelength in the range of from 350 to 850 nm.

9. The process according to any one of the preceding claims, wherein the wireless communication module (324) of the mobile digital device connects to the wireless communication module of the wearable control unit by using a Bluetooth protocol.

10. A garment (10) provided with an illumination system (20) for use in a process according to any one of the preceding claims, which comprises an illumination system (20) comprising
(a) an optical fibre member (210) holding fibre ends (212) of multiple optical fibres (211) attached to the garment (10);
(b) a wearable control unit (220) comprising at least one light source (221) releasably attached to the optical fibre member (210), the control unit further comprising a data processor (222), and a wearable power source (223);
**characterized in that** the light source (221) comprises an LED matrix having a number of LEDs (226), and the optical fibre member (210) comprises a plurality of fibres (211), whereby each fibre (211) is illuminated by a specific LED (226) of the light source (221) when the at least one light source (221) is releasably attached to the optical fibre member (210).

11. The garment (10) according to the preceding claim, wherein an LED (226) of the LED matrix illuminates a bundle of fibres (211).

12. The garment (10) according to claim 10 or 11, wherein the wearable control unit (220) further comprises one or more sensor modules (225) selected from an accelerometer sensor module, a gyroscope sensor module, a distance sensor module, a magnetometer sensor module, an orientation sensor module, a global positioning system module, a conductivity sensor module, an illuminance sensor module, and a microphone module.

13. A garment (10) comprising an optical fibre member (210) holding fibre ends (212) of at least two optical fibre bundles attached to the garment (210), wherein optical fibre member (210) is a plastic shell holding and arranging the optical fibre bundles as a n x m matrix, wherein each fibre bundle is independently composed of at least 3 optical fibres (211), and wherein n is an integer of 1 to 10 and m is an integer of from 2 to 10, so that the fibers are exposed to the inside of the shell for allowing light to enter the fibres (211).

14. An illumination system (20) for a garment (10), which comprises
(a) an optical fibre member (210) holding fibre ends (212) of one or more optical fibre bundles to be attached to the garment (10);
(b) a wearable control unit (220) comprising at least one light source (221) releasably attached to the optical fibre member (210), the control unit (220) further comprising a data processor (222), and a wearable power source (223), wherein the wearable control unit (220) further comprises
(b-1) a data collector configured to collect and store sensor data;
(b-2) a data processor configured to extract processed sensor data from the sensor data; and
(b-3) an individual state determiner configured to provide the processed sensor data to an inference model to determine the individual state based on the processed sensor data by using individual state rules.

15. The illumination system according to claim 14, which further comprises a wireless communication module.
